# EUROPÄISCHE PATENTSCHRIFT

(11) **EP 2 063 930 B1**
(45) Veröffentlichungstag und Bekanntmachung des Hinweises auf die Patenterteilung: **30.04.2014**
(21) Anmeldenummer: 07818081.7
(22) Anmeldetag: 08.09.2007
(51) Int. Cl.: A61M 1/36

(54) **VORRICHTUNG UND VERFAHREN ZUR ÜBERWACHUNG EINES ZUGANGS ZU EINEM PATIENTEN, INSBESONDERE EINES GEFÄSSZUGANGS BEI EINER EXTRAKORPORALEN BLUTBEHANDLUNG**
DEVICE AND METHOD FOR MONITORING A PATIENT ACCESS, IN PARTICULAR A VASCULAR ACCESS IN EXTRACORPOREAL BLOOD TREATMENT
DISPOSITIF ET PROCÉDÉ DE SURVEILLANCE D'UNE VOIE D'ACCÈS CHEZ UN PATIENT, EN PARTICULIER D'UNE VOIE D'ACCÈS VASCULAIRE DANS LE CAS D'UN TRAITEMENT SANGUIN EXTRACORPOREL

(30) Priorität: 12.09.2006 DE 102006042646
(43) Veröffentlichungstag der Anmeldung: 03.06.2009
(73) Patentinhaber: Fresenius Medical Care Deutschland GmbH, 61352 Bad Homburg v.d.H. (DE)
(72) Erfinder: KLEINEKOFORT, Wolfgang, 65779 Kelkheim (DE); WÜPPER, Andreas, 64572 Büttelborn (DE)
(74) Vertreter: Oppermann, Frank
(86) Internationale Anmeldenummer: PCT/EP2007/007849
(87) Internationale Veröffentlichungsnummer: WO 2008/031539

(56) Entgegenhaltungen:
- WO-A-03/086505
- DE-B3-102006 041 265
- DE-C1- 19 739 099
- US-A1- 2003 195 453

## Beschreibung

Die Erfindung betrifft Blutbehandlungsvorrichtung mit einer Vorrichtung zur Überwachung eines Zugangs zu einem Patienten, und ein Verfahren zur Überwachung eines Gefäßzugangs bei einer extrakorporalen Blutbehandlung, bei der Blut eines Patienten über eine arterielle Schlauchleitung, die einen arteriellen Patientenanschluss mit einer arteriellen Punktionskanüle aufweist, dem Patienten entnommen und über eine venöse Schlauchleitung, die über einen venösen Patientenanschluss mit einer venösen Punktionskanüle aufweist, dem Patienten wieder zugeführt wird.

Auf dem Gebiet der Medizintechnik sind eine Vielzahl von Einrichtungen bekannt, mit denen über eine Schlauchleitung Patienten Flüssigkeiten entnommen oder Flüssigkeiten den Patienten zugeführt werden können. Dabei erfolgt der Zugang zu den Patienten im Allgemeinen mit Kathetern zum Einführen in Körperorgane oder Kanülen zum Punktieren von Gefäßen. Während der Untersuchung oder Behandlung ist ein ordnungsgemäßer Zugang zu dem Patienten sicherzustellen. Daher ist es erforderlich, den Patientenzugang zu überwachen.

Bei Verfahren der Blutreinigungstherapie wie Hämodialyse, Hämofiltration und Hämodiafiltration wird Blut eines Patienten über einen extrakorporalen Blutkreislauf geleitet, in dem sich ein Membranfilter, z.B. ein Dialysator oder ein Hämofilter oder Hämodiafilter befindet, der durch eine semipermeable Membran in eine Blutkammer und eine davon getrennte Dialysierflüssigkeitskammer bzw. Filtratkammer, über die der Filtratabfluss erfolgt, aufgeteilt ist. Bei einer Hämodiafiltrationsmaschine, bei der sowohl Hämodialyse als auch Hämofiltration simultan durchgeführt werden, erfolgt auf der Dialysatseite gleichzeitig auch der Filtratabfluss. Falls sich der venöse Patientenanschluss während der Blutbehandlung löst, kann ein Verbluten des Patienten nur verhindert werden, wenn innerhalb weniger Sekunden der extrakorporale Blutfluss gestoppt wird. Daher ist der extrakorporale Blutkreislauf der bekannten Blutbehandlungsvorrichtungen im Allgemeinen mit Schutzsystemen ausgestattet, die im Alarmfall die Blutpumpe stoppen, die venöse Schlauchklemme schließen sowie ein akustisches und/oder optisches Warnsignal geben.

Die DE 197 39 099 C1 beschreibt eine Vorrichtung zur Überwachung eines Gefäßzugangs während einer extrakorporalen Blutbehandlung, bei der ein elektrischer Strom in der eine geschlossene Leiterschleife darstellenden Verbindung des extrakorporalen Blutkreislaufs induziert wird, der in der Leiterschleife fließende Strom gemessen und bei einer charakteristischen Veränderung der Stromstärke auf einen fehlerhaften Gefäßzugang geschlossen wird. Neben der induktiven Ein- und Auskopplung ist aus der US 6,932,786 B2 bekannt, elektrische Signale in den extrakorporalen Blutkreislauf kapazitiv ein- und auszukoppeln.

Die WO 99/29356 beschreibt eine Vorrichtung zur Überwachung eines Gefäßzugangs mit einer Punktionskanüle, an der eine Schlauchleitung angeschlossen ist, die zu einer Flüssigkeitsquelle führt. Zur Überwachung des Gefäßzugangs wird stromab der Flüssigkeitsquelle an einer ersten Stelle der Schlauchleitung ein elektrisches Signal in den Flüssigkeitsstrom eingekoppelt und an einer zweiten Stelle stromab der ersten Stelle ein elektrisches Signal aus dem Flüssigkeitsstrom ausgekoppelt. Das elektrische Signal kann aber auch an einer auf der Haut des Patienten befindlichen Stelle ausgekoppelt werden. Wenn die ausgekoppelten elektrischen Signale größer als ein vorgegebener Grenzwert sind, wird ein Alarm ausgelöst.

Die US 2003/0195454 A1 setzt sich mit der Problematik der kapazitiven Ein- und Auskopplung von Messsignalen in den extrakorporalen Blutkreislauf auseinander und schlägt vor, die Messsignale mittels elektrischer Kontaktelemente, die mit dem durch die Schlauchleitungen strömenden Blut in Kontakt stehen, in den extrakorporalen Kreislauf ein- und auszukoppeln.

Aus der US 2006/0081517 A1 ist eine Vorrichtung zur Überwachung des arteriellen und venösen Gefäßzugangs bei einer extrakorporalen Blutbehandlung mit einer Hämodialysevorrichtung bekannt, bei der eine vorgegebene Spannung an den Dialysierflüssigkeits- und Blutkreislauf angelegt wird. Die Spannung liegt zwischen zwei Elektroden an, von denen die eine mit dem im Blutkreislauf strömenden Blut und die andere mit der im Dialysierflüssigkeitskreislauf strömenden Dialysierflüssigkeit in Kontakt steht, so dass im Blut- und Dialysierflüssigkeitskreislauf ein elektrischer Strom fließt. Dabei setzt die bekannte Vorrichtung voraus, dass die peristaltische Blutpumpe, die im Blutkreislauf angeordnet ist, eine Unterbrechung des Stromkreises darstellt.

Die oben beschriebenen Vorrichtungen und Verfahren haben den Nachteil, dass elektrische Signale in die strömenden Flüssigkeiten eingekoppelt werden müssen, wodurch sich der Aufwand für die Überwachung des Patientenzugangs erhöht. Darüber hinaus hat sich die Einkopplung der elektrischen Signale in den Flüssigkeitsstrom vielfach als problematisch erwiesen.

Der Artikel von Ross et al., Kidney International (2006) 69, 2274-2277 beschreibt ein Verfahren zur Überwachung eines Patientenzugangs, bei dem elektrische Messsignale nicht in den Flüssigkeitsstrom eingekoppelt werden, sondern ausschließlich endogene (körpereigene) elektrische Spannungen ausgewertet werden. Das Verfahren von Ross et al. setzt die Herstellung einer elektrischen Verbindung von zwei Elektroden mit dem Blut voraus. Die körpereigenen elektrischen Signale, die an der venösen und arteriellen Schlauchleitung abgegriffen werden, sind auf Grund der nur wenige Zentimeter messenden Entfernung der Messelektroden nahezu identisch. Bei einem ordnungsgemäßen Patientenzugang ist die Differenz der gemessenen Signale etwa Null. Bei einem fehlerhaften Gefäßzugang hingegen kann eine Potentialdifferenz nachgewiesen werden.

Das bekannte Verfahren beruht also auf der Überwachung der Potentialdifferenz von körpereigenen Signalen, wobei die Einkopplung elektrischer Messsignale in den Flüssigkeitsstrom nicht erforderlich ist. Eine Erkennung von EKG (Elektrokardiogramm)-Signalen mit einer Spannungsmessung an den eng nebeneinanderliegenden Messpunkten dürfte mit dem bekannten Verfahren aber kaum möglich sein, weil der sogenannte "Herzvektor" bei einem derart engen Abstand der Elektroden nicht ausreichend erfasst werden kann. Daher dürften zwangsläufig mit den Messelektroden nur diffuse körpereigene Signale erfasst werden können. Zwangsläufig werden bei der Messung auch Störsignale aus der Umgebung gemessen, beispielsweise Brummspannungen (50 Hz bzw. 60 Hz), die der Netzspannung überlagert sind.

Die WO 2004/108206 A1 setzt sich mit dem Problem auseinander, dass okkludierende Blutpumpen Störsignale erzeugen, die körpereigene Signale überlagern. Dieses Problem stellt sich insbesondere bei der Messung von EKG-Signalen, während die Blutpumpe einer extrakorporalen Blutbehandlungsvorrichtung läuft. Aus der WO 2004/108206 A1 ist weiterhin bekannt, dass der Dialysator einer Hämodialysevorrichtung keine Barriere für einen elektrischen Stromfluss zwischen Blut- und Dialysierflüssigkeitskreislauf darstellt.

Der Artikel von Jos Snijders in Perfusion 1999; 14: 363-370 befasst sich mit den Ursachen der elektrostatischen Aufladung in extrakorporalen Blutkreisläufen sowie deren Prävention, während RJ Elgas in Perfusion 1999; ; 14: 133-140 sich mit dem Phänomen der elektrostatischen Aufladung in einem Wärmetauscher mit Kunststofffasern befasst.

Der Erfindung liegt die Aufgabe zu Grunde eine Blutbehandlungsvorrichtung mit einer Vorrichtung bereitzustellen, die eine Überwachung des Patientenzugangs mit hoher Sicherheit ohne die Einkopplung von Messsignalen ermöglicht. Des weiteren ist es eine Aufgabe der Erfindung, ein Verfahren anzugeben, das die Überwachung des Zugangs mit hoher Sicherheit ohne Einkopplung eines Messsignals erlaubt.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung und das erfindungsgemäße Verfahren unterscheiden sich von denen aus dem Stand der Technik bekannten Überwachungsvorrichtungen bzw. -verfahren dadurch, dass die für die Messung erforderlichen elektrischen Signale, die einen Stromfluss in der Flüssigkeit bewirken, die in den Schlauchleitungen strömt, nicht extern eingekoppelt werden müssen, sondern von der Pumpe oder den Pumpen zum Fördern der Flüssigkeit erzeugt werden. Daher sind bei dem erfindungsgemäßen Verfahren bzw. der erfindungsgemäßen Vorrichtung externe Einrichtungen zur Erzeugung von Messsignalen nicht erforderlich, wodurch sich der apparative Aufwand verringert.

Die als tribo-elektrische Spannungen bekannten "Störsignale", die von der im extrakorporalen Blutkreislauf angeordneten okkludierenden Blutpumpe erzeugt werden, können vorteilhaft zur Erkennung einer Nadeldiskonnektion und/oder einer Nadeldislokation der venösen und/oder arteriellen Punktionskanüle verwendet werden, so dass sich die Nachteile der bekannten Verfahren, die auf der Erfassung körpereigener elektrischer Signale basieren, überwunden werden können.

Eine Grundvoraussetzung der erfindungsgemäßen Vorrichtung bzw. des erfindungsgemäßen Verfahrens ist, dass die Flüssigkeit mit einer oder mehreren Pumpen gefördert wird, die eine tribo-elektrische Wechselspannung erzeugen. Dies sind vor allem die okkludierenden Schlauchpumpen, die in Blutbehandlungsvorrichtungen im Allgemeinen Verwendung finden. Unter einer okkludierenden Pumpe ist eine Pumpe zu verstehen, deren Pumpwirkung darauf basiert, dass sich mindestens eine Eng- oder Verschlussstelle (Okklusion) längs des als Pumpraum dienenden elastischen Schlauchs bewegt und dadurch die eingeschlossene Flüssigkeit in Förderrichtung verschiebt. Die Eng- bzw. Verschlussstellen können beispielsweise durch Rollen oder Finger oder dgl. erzeugt werden. Die Okklusion des Pumpenschlauchsegments führt zu einer galvanischen Trennung des Stromkreises, der mit der durch die Schlauchleitungen strömenden Flüssigkeit gebildet wird.

Das erfindungsgemäße Verfahren bzw. die erfindungsgemäße Vorrichtung machen also von dem Phänomen Gebrauch, das bei peristaltischen Schlauchpumpen unter dem Begriff Tribo-Elektrizität bekannt ist. Bei peristaltischen Pumpen ist bekannt, dass durch die Rollen der Rollenpumpe Ladungstrennungen an der inneren und äußeren Schlauchoberfläche auftreten. Bei okkludierenden Rollenpumpen führen die Ladungstrennungen zu elektrischen Aufladungen sowohl an der Schlauchaußenseite und den Rollen als auch an der Schlauchinnenseite, wobei die Ladungen von der Schlauchaußenseite und den Rollen abfließen können, da die Schlauchaußenseite und die Rollen im Allgemeinen geerdet sind. Bisher sind die tribo-elektrischen Spannungen aber nur als Störfaktoren angesehen worden.

Aufgrund der tribo-elektrischen Spannung kommt es in der Flüssigkeit zu einem Stromfluss, wobei die Pumpe als Spannungsgenerator fungiert. Bei einem fehlerhaften Gefäßzugang, beispielsweise einer Diskonnektion der arteriellen und/oder venösen Kanüle, wird der Stromfluss unterbrochen.

Die erfindungsgemäße Vorrichtung bzw. das erfindungsgemäße Verfahren sehen vor, dass die Amplitude der gemessenen tribo-elektrischen Wechselspannung überwacht wird, wobei auf einen fehlerhaften Gefäßzugang dann geschlossen wird, wenn sich die Amplitude der gemessenen Spannung innerhalb vorgegebener Grenzen verändert. Es kann aber auch eine Messung des tribo-elektrischen Wechselstroms oder einer abgeleiteten elektrischen Messgröße, z. B. des Widerstandes erfolgen.

Die tribo-elektrische Wechselpannung wird bei einer Hämodialysemaschine zwischen der Haut des Patienten und der im Dialysierflüssigkeitskreislauf strömenden Dialysierflüssigkeit gemessen. Daher ist es nicht erforderlich, Messabgriffe an der arteriellen und/oder venösen Punktionskanüle bzw. dem extrakorporalen Blutkreislauf vorzusehen, die sich in der Praxis als kritisch erwiesen haben. Die tribo-elektrische Wechselspannung wird zwischen einem elektrischen Kontaktelement gemessen, das auf die Haut des Patienten aufgelegt wird, und einem weiteren Kontaktelement, das mit der im Dialysierflüssigkeitskreislauf strömenden Dialysierflüssigkeit in Kontakt steht. Dies ist deshalb möglich, weil der Dialysierflüssigkeitskreislauf über die semipermeable Membran des Dialysators mit dem Blutkreislauf elektrisch verbunden ist. Analog kann die tribo-elektrische Spannung bei einer Hämofiltrationsmaschine oder einer Hämodiafiltrationsmaschine auch zwischen der Haut des Patienten und dem Filtrat gemessen werden, weil die Filtratseite des Hämofilters oder des Hämodiafilters über die semipermeable Membran mit dem Blutkreislauf elektrisch verbunden ist. Grundsätzlich ist es aber auch möglich, anstelle der Messung des Potentials gegenüber einer an dem Patienten anzubringenden Referenzelektrode eine Messung des Potentials gegenüber Masse, d.h. der Betriebserde der Blutbehandlungsvorrichtung vorzusehen. Dies ist nicht Gegenstand der Erfindung.

Als elektrisches Kontaktelement, das an einer beliebigen Stelle der Hautoberfläche des Patienten anzubringen ist, kann beispielsweise eine standardisierte EKG-Elektrode eingesetzt werden.

Bei einer bevorzugten Ausführungsform wird die Amplitude der gemessenen tribo-elektrischen Wechselspannung mit einem vorgegebenen Grenzwert verglichen, wobei auf einen fehlerhaften Gefäßzugang dann geschlossen wird, wenn die Amplitude der Wechselspannung größer als der vorgegebene Grenzwert ist. Es ist grundsätzlich auch ein Vergleich mit mehreren vorgegebenen Grenzwerten möglich, um zwischen unterschiedlichen Zuständen unterscheiden zu können.

Zur Erhöhung der Sicherheit wird die Amplitude der Wechselspannung zu verschiedenen aufeinanderfolgenden Zeitpunkten mit einem vorgegebenen Grenzwert verglichen. Auf einen fehlerhaften Gefäßzugang wird dann geschlossen, wenn für eine vorgegebene Anzahl von Messungen zu verschiedenen aufeinanderfolgenden Zeitpunkten die Amplitude der Wechselspannung größer als der vorgegebene Grenzwert ist. Je größer die Anzahl der aufeinanderfolgenden Messungen ist, je größer ist die Sicherheit, mit der ein fehlerhafter Gefäßzugang erkannt wird. Andererseits ist die Empfindlichkeit größer, wenn die Anzahl der Messungen kleiner ist.

Die Sicherheit der Messung kann weiterhin dadurch erhöht werden, dass die Frequenz des elektrischen Wechselspannungssignals gemessen und mit der Frequenz des Rotors der Pumpe verglichen wird. Bei einer Rollenpumpe beispielsweise hängt die Frequenz des Wechselspannungssignals von der Drehzahl des Rotors und der Anzahl der Rollen ab. Auf einen fehlerhaften Gefäßzugang wird nur dann geschlossen, wenn die Frequenz der Wechselspannung und die Frequenz des Rotors der Schlauchpumpe miteinander korrelieren. Andernfalls wird davon ausgegangen, dass nicht die tribo-elektrische Spannung, sondern ein anderes Störsignal gemessen wird, das keinen Schluss auf einen fehlerhaften Gefäßzugang zulässt.

Die erfindungsgemäße Blutbehandlungsvorrichtung und das erfindungsgemäße Verfahren zum Überwachen des Zugangs bei einer extrakorporalen Blutbehandlung sehen vor, dass die tribo-elektrische Wechselspannung und/oder der tribo-elektrische Wechselstrom und/oder eine davon abgeleitete elektrische Messgröße auf Grund von elektrostatischen Aufladungen beim Betrieb der Pumpe überwacht wird.

Im Folgenden wird ein Ausführungsbeispiel der Erfindung unter Bezugnahme auf die Zeichnungen näher erläutert:

Es zeigen:
- Fig. 1: Die wesentlichen Komponenten einer Blutbehandlungsvorrichtung zusammen mit einer Vorrichtung zur Überwachung des Patientenzugangs in stark vereinfachter schematischer Darstellung,
- Fig. 2a und 2b: Ersatzschaltbilder der Blutbehandlungsvorrichtung und des Patienten bei einem ordnungsgemäßen und einem fehlerhaften Gefäßzugang,
- Fig. 3: einen Flusslaufplan des erfindungsgemäßen Verfahrens,
- Fig. 4: einen Versuchsaufbau zur Simulation des erfindungsgemäßen Verfahrens und
- Fig. 5: die bei dem Versuchsaufbau von Fig. 4 gemessene tribo-elektrische Spannung als Funktion der Zeit während eines ordnungsgemäßen und eines fehlerhaften Gefäßzugangs.

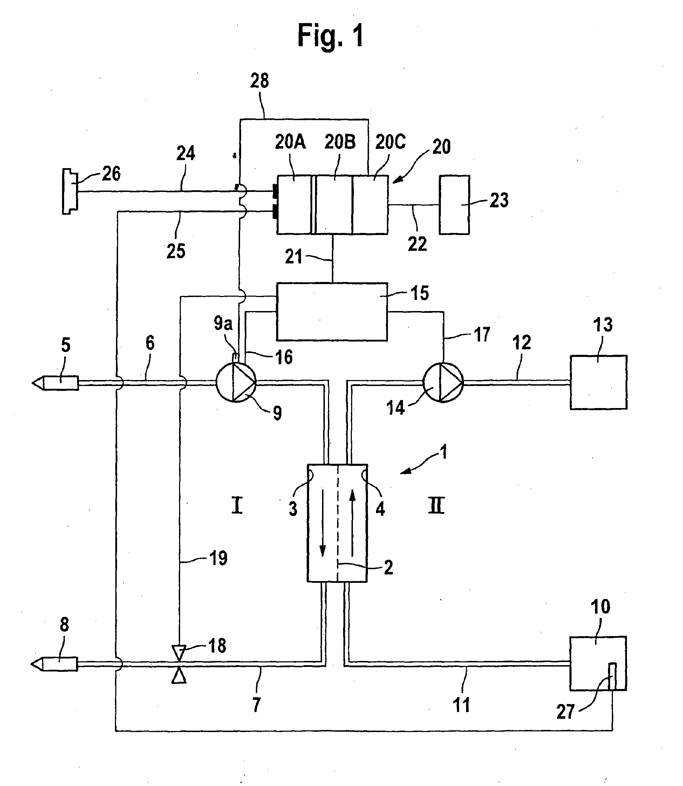

Fig. 1 zeigt die wesentlichen Komponenten einer Hämodialysevorrichtung, die über eine Vorrichtung zur Überwachung des arteriellen und/oder venösen Gefäßzugangs verfügt. Die Hämodialysevorrichtung weist einen Dialysator 1 auf, der durch eine semipermeable Membran 2 in eine Blutkammer 3 und eine Dialysierflüssigkeitskammer 4 unterteilt ist. An einer Arterie des Patienten ist mittels einer arteriellen Punktionskanüle 5 eine flexible arterielle Schlauchleitung 6 angeschlossen, die zu dem Einlass der Blutkammer 3 des Dialysators 1 führt. Von dem Auslass der Blutkammer 3 des Dialysators 1 geht eine flexible venöse Schlauchleitung 7 ab, die mittels einer venösen Punktionskanüle 8 an einer Vene des Patienten angeschlossen ist. Die arterielle Schlauchleitung 6 ist in eine okkludierende Blutpumpe 9, insbesondere eine peristaltische Rollenpumpe eingelegt, die das Blut im extrakorporalen Blutkreislauf I fördert.

Der Dialysierflüssigkeitskreislauf II der Hämodialysevorrichtung umfaßt eine Dialysierflüssigkeitsquelle 10, an der eine Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet. Für den Fall der Hämofiltration stellt die "Dialysierflüssigkeitskammer" eine Filtratkammer 4 und die "Dialysierflüssigkeitsabführleitung" eine Filtratabführleitung 12 dar, über die der Filtratabfluss erfolgt.

Die Steuerung der Dialysevorrichtung übernimmt eine zentrale Steuereinheit 15, die über Steuerleitungen 16, 17 die Blut- und Dialysierflüssigkeitspumpe 9, 14 ansteuert. Stromab der Blutkammer 3 des Dialysators 1 befindet sich an der venösen Schlauchleitung 7 eine elektromagnetisch betätigbare Schlauchklemme 18, die über eine weitere Steuerleitung 19 von der zentralen Steuereinheit 15 geschlossen wird, wenn die venöse Punktionskanüle 8 (Nadel) aus dem Gefäßzugang herausrutschen sollte. Darüber hinaus stoppt die Steuereinheit 15 nach dem Herausrutschen der Kanüle die Blutpumpe 9.

Zur Überwachung des arteriellen und venösen Gefäßzugangs weist die Dialysevorrichtung eine Überwachungsvorrichtung 20 auf, die über eine Datenleitung 21 mit der zentralen Steuereinheit 15 kommuniziert. Die Überwachungseinrichtung 20 ist über eine weitere Datenleitung 22 mit einer Alarmeinheit 23 verbunden, die für den Fall eines fehlerhaften Gefäßzugangs, beispielsweise einer Diskonnektion oder einer Dislokation der venösen Punktionskanüle 8 einen optischen und/oder akustischen Alarm gibt. Weiterhin steuert die Überwachungseinrichtung 20 für den Fall eines fehlerhaften Gefäßzugangs die zentrale Steuereinheit 15 an, die dann die venöse Schlauchklemme 18 schließt und die Blutpumpe 9 stoppt.

Nachfolgend wird der Aufbau und die Funktionsweise der erfindungsgemäßen Vorrichtung beschrieben.

Die Überwachungsvorrichtung 20 verfügt über Mittel 20A zum Messen und Filtern einer im vorliegenden Ausführungsbeispiel Wechselspannung mit einem sehr hochohmigen Eingang, an dem über Messleitungen 24, 25 ein erstes elektrisches Kontaktelement 26 und ein zweites elektrisches Kontaktelement 27 angeschlossen sind, zwischen denen eine Wechselspannung gemessen wird. Der Messbereich der Messeinheit 20A für das gefilterte Messsignal liegt zwischen 0 und 2 V, vorzugsweise 0 bis 1 V, insbesondere 0 bis 500 mV. Das ungefilterte Messsignal hat einen Pegel von ca. 7 V und kann in Abhängigkeit von den Umgebungsbedingungen auch höhere Werte annehmen, weil Elektrogeräte in der Umgebung, speziell im Heimbereich, wo Elektrogeräte nicht abgeschirmt sind, als Signalquelle wirken können. Die Auslegungswerte der Messeinheit 20A können deshalb z.B. 15 V oder auch 30 V betragen. Auch für das gefilterte Signal können höhere Werte als 7 V auftreten. Bei der Implementierung der Vorrichtung in eine Blutbehandlungsvorrichtung wird zwischen dem Spannungsmessgerät und dem Patienten eine Schutzvorrichtung gegen eine Übertragung von Überspannungen auf den Patienten vorgesehen. Diese kann beispielsweise durch zusätzliche Schutzimpedanzen realisiert werden, was zu höheren Spannungswerten führt.

Das erste elektrische Kontaktelement 26 ist eine konventionelle EKG-Elektrode, die beispielsweise am Handgelenk des Patienten befestigt wird, während das zweite elektrische Kontaktelement 27 sich mit der Dialysierflüssigkeit elektrisch in Kontakt befindet, beispielsweise in der Einrichtung 10 zur Bereitstellung der Dialysierflüssigkeit angeordnet ist, so dass die auf Grund von elektrostatischen Aufladungen beim Betrieb der okkludierenden Blutpumpe 9 erzeugte elektrische Wechselspannung gemessen werden kann.

Zur Erfassung der tribo-elektrischen Wechselspannung könnte grundsätzlich auch eine erste Elektrode an der arteriellen Blutleitung 6 und eine zweite Elektrode an der venösen Blutleitung 7 angeordnet sein, die mit dem im arteriellen oder venösen Zweig des extrakorporalen Blutkreislaufs I strömenden Blut in Berührung kommen. Der Kontakt des Bluts mit Elektroden oder dgl. sollte aber grundsätzlich vermieden werden. Hinzu kämen höhere Herstellungskosten für das Schlauchset. Die erfindungsgemäße Vorrichtung hingegen verzichtet auf Elektroden, die im arteriellen und venösen Zweig des extrakorporalen Kreislaufs angeordnet sind und sieht zwei elektrische Kontaktelemente vor, von denen das eine auf die Haut des Patienten aufgelegt wird und das andere nicht mit dem Blut, sondern der Dialysierflüssigkeit in Kontakt steht. Das zweite elektrische Kontaktelement kann jedes leitende Element sein, das mit der im Dialysierflüssigkeitskreislauf strömenden Dialysierflüssigkeit Kontakt hat. Beispielsweise können die Metallflansche von sogenannten Hansenkupplungen oder die Elektroden eines im Dialysierflüssigkeitskreislauf angeordneten Niveaufühlers das zweite elektrische Kontaktelement bilden.

Der Potentialabgriff unter Verwendung des ersten elektrischen Kontaktelements 26, das auf die Haut des Patienten aufgelegt wird, kann deshalb vorgenommen werden, weil die arterielle Punktionskanüle 5 über den Gefäßzugang mit dem kardiovaskulären Gefäßsystem des Patienten in leitender Verbindung steht. Der Widerstand R zwischen Gefäßsystem des Patienten und Hautoberfläche beträgt 1-2 MΩ, so dass Potentialänderungen innerhalb des Gefäßsystems an der Hautoberfläche direkt nachgewiesen können. Dabei spielt die Position, mit der das erste elektrische Kontaktelement an der Haut des Patienten angebracht wird, grundsätzlich keine Rolle.

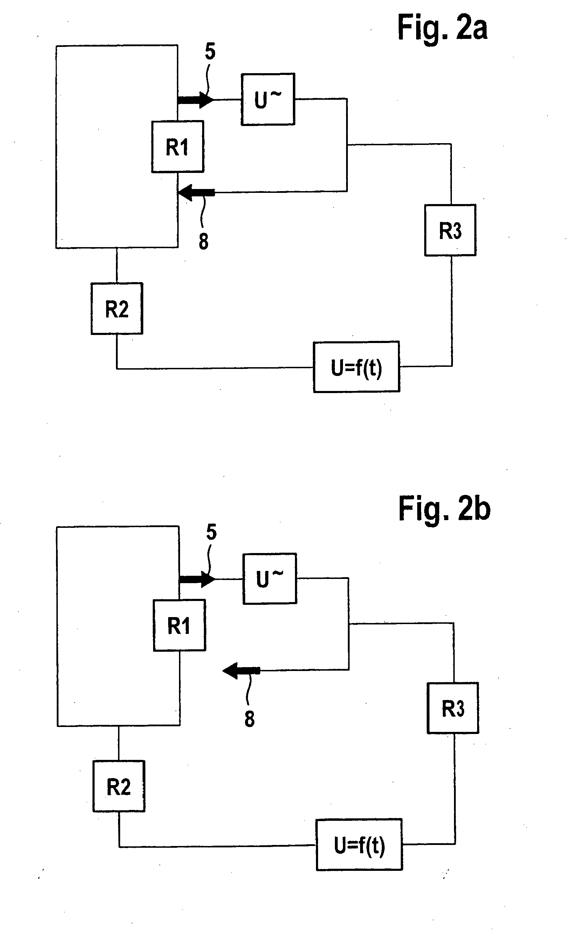

Fig. 2a zeigt ein vereinfachtes Ersatzschaltbild des Patienten und der Dialysevorrichtung. Die okkludierende Blutpumpe 9 der Dialysevorrichtung ist in dem Ersatzschaltbild als Generator U dargestellt, der eine Wechselspannung erzeugt. R1 bezeichnet den Widerstand zwischen arterieller und venöser Punktionskanüle 5, 8. R2 ist der Widerstand zwischen dem kardiovaskulären Gefäßsystem und der Hautoberfläche, und R3 ist der Widerstand zwischen dem zweiten Kontaktelement 27 im Dialysierflüssigkeitskreislauf II und dem Dialysator 1. In dem vereinfachten Ersatzschaltbild ist der Dialysator, der einen zu vernachlässigenden Widerstand hat, nicht dargestellt. Ebenso sind zur Vereinfachung die Widerstände im kardiovaskulären Gefäßsystem nicht dargestellt. Die Überwachungseinrichtung 20 mit den Mitteln 20A zum Messen des zeitlichen Verlaufs der tribo-elektrischen Wechselspannung ist mit U=f(t) bezeichnet. Figur 2a zeigt den Fall, dass sowohl die arterielle als auch venöse Punktionskanüle 5, 8 ordnungsgemäß mit dem Gefäßsystem des Patienten verbunden sind.

Fig. 2b zeigt den Fall, dass die venöse Punktionskanüle 8 aus dem Gefäßsystem des Patienten herausgerutscht ist. Ansonsten unterscheiden sich die Figuren 2a und 2b nicht.

Für den Fall eines ordnungsgemäßen Gefäßzugangs gilt R1 << R2. Da über die Messeinheit 20A, die einen sehr hochohmigen Eingang hat, nur ein zu vernachlässigender Strom fließt, führt die von der okkludierenden Blutpumpe 9 erzeugte Spannung dazu, dass ein Strom über den Widerstand R1 fließt. Zwischen Hautoberfläche R2 und Dialysierflüssigkeitskreislauf I sind nur kleine Spannungsamplituden messbar.

Wenn die venöse Punktionskanüle hingegen aus dem Gefäßzugang herausrutscht (Fig. 2b), wird der Widerstand R1 zwischen dem arteriellen und venösen Zweig des extrakorporalen Blutkreislaufs I unendlich groß (R1 >> R2), so dass die von der Blutpumpe 9 erzeugte tribo-elektrische Wechselspannung als Potentialdifferenz zwischen der Hautoberfläche und der im Dialysierflüssigkeitskreislauf II strömenden Dialysierflüssigkeit gemessen werden kann.

Die in Fig. 1 dargestellte Dialysevorrichtung mit der Vorrichtung zur Überwachung des Gefäßzugangs arbeitet wie folgt.

Die Messeinheit 20A der Überwachungsvorrichtung 20 misst mit den Kontaktelementen 26, 27 die Potentialdifferenz zwischen der Hautoberfläche des Patienten und der im Dialysierflüssigkeitskreislauf II strömenden Dialysierflüssigkeit. Die Überwachungsvorrichtung 20 verfügt weiterhin über eine Auswerteinheit 20B und 20C, die ihrerseits eine Vergleichseinheit 20B zum Vergleichen der Amplitudenwerte A der gemessenen Wechselspannung mit einem vorgegebenen Grenzwert B(f) und eine Vergleichseinheit 20C zum Vergleichen der Rotorfrequenz der Blutpumpe 9 mit der Frequenz f der gemessenen Wechselspannung aufweist. Die Vergleichseinheit 20C ist über eine Leitung 28 mit der Blutpumpe 9 verbunden, um das Signal des in der Pumpe integrierten Hall-Sensors 9a zur Bestimmung der Rotorfrequenz f der Pumpe erfassen zu können. Da die Amplitude der gemessenen Wechselspannung von der Rotorfrequenz f der Blutpumpe abhängt, wird der vorgegebene Grenzwert B(f) als Funktion der Rotorfrequenz f gewählt.

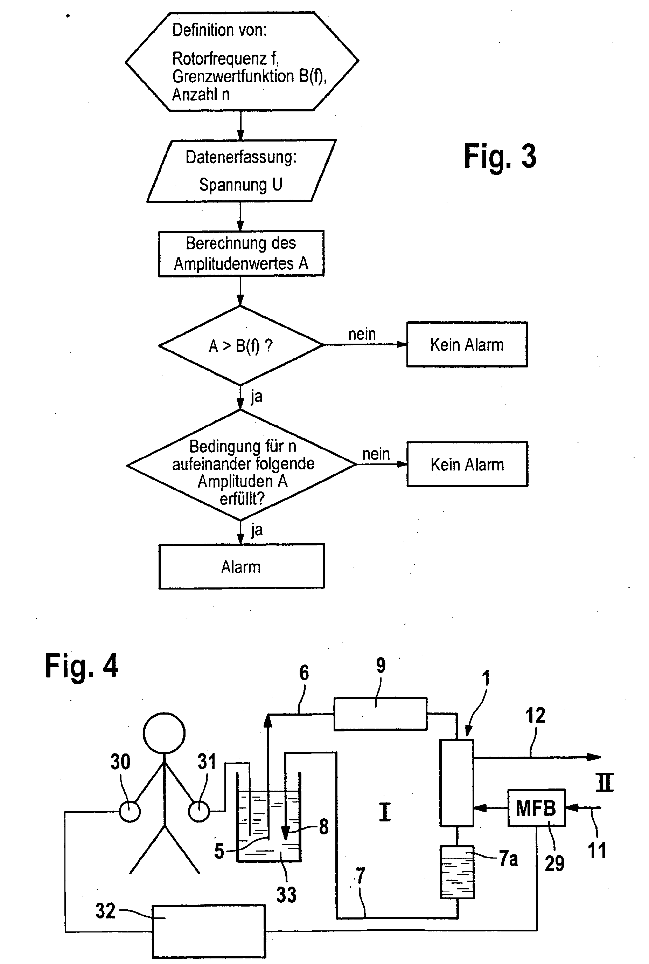

Fig. 3 zeigt den Flusslaufplan der Auswertung der Messsignale. Für den Fall, dass die Werte der Amplitude des gemessenen Wechselspannungssignals für n aufeinanderfolgende Amplitudenwerte den Grenzwert B(f) überschreiten, erzeugt die Überwachungseinrichtung 20 ein Alarmsignal. Ein fehlerhafter Gefäßzugang wird nach dem Erzeugen des Alarmsignals aber nur dann angenommen, wenn die Rotorfrequenz der Blutpumpe 9 mit der Frequenz des gemessenen Wechselspannungssignals korrelieren. Hierzu vergleicht die Vergleichseinheit 20C beide Frequenzen. Wenn beide Frequenzen in einem vorgegebenen Verhältnis zueinander stehen, was von der Anzahl der Rollen der Rollenpumpe 9 abhängt, wird darauf geschlossen, dass das gemessene Signal tatsächlich das auf Grund von elektrostatischen Aufladungen beim Betrieb der Blutpumpe erzeugte tribo-elektrische Spannungssignal, nicht aber ein anderes "Störsignal" ist. Nur für diesen Fall wird angenommen, dass ein fehlerhafter Gefäßzugang vorliegt und das Alarmsignal wird an die Alarmeinheit 23 und die zentrale Steuereinheit 15 ausgegeben, so dass die Alarmeinheit einen akustischen und/oder optischen Alarm gibt und die Steuereinheit die venöse Schlauchklemme 18 schließt und die Blutpumpe 9 stoppt.

Nachfolgend wird unter Bezugnahme auf Fig. 4 das Ergebnis der Messung der tribo-elektrischen Wechselspannung mit einem experimentellen Versuchsaufbau beschrieben. Dabei werden die Teile des Versuchsaufbaus, die denjenigen Teilen der unter Bezugnahme auf Figur 1 beschriebenen Dialysevorrichtung entsprechen, mit den gleichen Bezugszeichen versehen.

Der Versuchsaufbau umfaßt ein Dialysator 1 mit einem Blutkreislauf I und einem Dialysierflüssigkeitskreislauf II. Der Blutkreislauf I umfaßt eine arterielle Blutleitung 6, in der eine okkludierende Blutpumpe 9 angeordnet ist und eine venöse Blutleitung 7 mit einer venösen Tropfkammer 7a, während der Dialysierflüssigkeitskreislauf II eine Dialysierflüssigkeitszuführleitung 11 und eine Dialysierflüssigkeitsabführleitung 12 aufweist. Die Dialysierflüssigkeitszuführleitung 11 führt durch einen sogenannten Multifunktionsblock (MFB) 29 der zu den Testzwecken eingesetzten Dialysevorrichtung.

Eine Testperson wurde im Bereich des rechten und linken Unterarms mit EKG-Elektroden 30, 31 versehen. Eine der Elektroden 30 wurde mit Messkanal 1 einer Spannungsmesseinrichtung 32 verbunden, wobei Messkanal 2 direkt mit der Dialysierflüssigkeit verbunden wurde. Hierzu wurde der Messkanal 2 über ein Kabel mit dem Kontaktstecker des Niveaufühlers am Multifunktionsblock (MFB) 29 verbunden. Die zweite Elektrode 31 wurde über ein Kabel mit der in einem Becherglas 33 befindlichen Dialysierflüssigkeit verbunden, das als Flüssigkeitsreservoir diente. Als Blutersatzflüssigkeit wurde Dialysierflüssigkeit verwendet. Um eine leitfähige Verbindung zwischen Elektrode, Kanüle, Schlauchset und Dialysator zu erhalten, wurde das Schlauchset bis zur Maximalhöhe der venösen Tropfkammer 7a mit physiologischer Kochsalzlösung gefüllt. Die Leitfähigkeit betrug 14,3 mS/cm. Auf der Dialysatseite wurde eine Standard-Dialysierflüssigkeit mit identischer Leitfähigkeit gewählt. Die arterielle und venöse Punktionskanüle 5, 8 befanden sich im Becherglas 33. Für alle Versuche wurden Standard-Schlauchsets und -Kanülen verwendet.

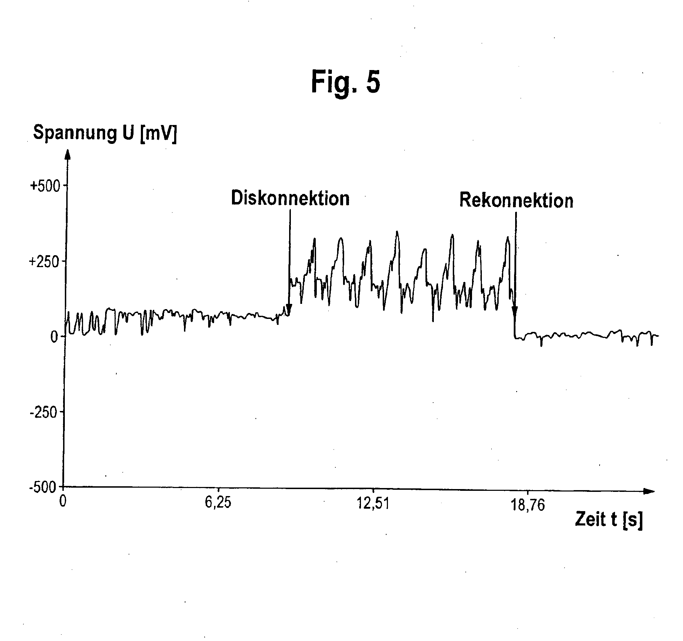

Fig. 5 zeigt exemplarisch den zeitlichen Verlauf der Spannungsmessung zwischen Dialysierflüssigkeitskreislauf II und EKG-Elektrode 30 am Handgelenk der Testperson. Der Blutfluss betrug 300 ml/min und der Dialysatfluss war 300 ml/min. Anschließend wurde die venöse Kanüle aus dem Becherglas genommen. Man sieht, dass sich das Signal ohne Zeitverzögerung verändert hat. Bei Eintauchen der Kanüle in das Becherglas ist das ursprüngliche Signal wieder zu erkennen.

Die Oszillation im Signal nach venöser Diskonnektion entspricht der doppelten Umdrehungsfrequenz des Rotors der Blutpumpe. Der Datensatz wurde unter Verwendung eines FIR Low-Pass-Filters mit einer Cut-Off-Frequenz von 10 Hz aufbereitet. In der Folge wurden Testreihen unter Variation des Blut- und Dialysatflusses aufgenommen. In allen Testreihen mit Blutflüssen zwischen 100 ml/min und 600 ml/min und Dialysatflüssen von 300, 500 und 800 ml/min konnte eine venöse Nadeldiskonnektion eindeutig erkannt werden.

## Patentansprüche

1. Blutbehandlungsvorrichtung mit einem
Dialysierflüssigkeitskreislauf (II), der eine Dialysierflüssigkeitskammer (4) eines von einer semipermeablen Membran (2) in eine Blutkammer (3) und die Dialysierflüssigkeitskammer (4) unterteilten Membranfilters (1) einschließt und/oder einem Filtratablauf, der eine Filtratkammer (4) eines von einer semipermeablen Membran (2) in eine Blutkammer (3) und die Filtratkammer (4) unterteilten Membranfilters (1) einschließt,
einem Blutkreislauf (I), der die Blutkammer des Dialysators einschließt, wobei eine arterielle Schlauchleitung (6), die an einer arteriellen Punktionskanüle (5) angeschlossen ist, zu der Blutkammer des Membranfilters führt und eine venöse Schlauchleitung (7), die an einer venösen Punktionskanüle (8) angeschlossen ist, von der Blutkammer des Membranfilters abgeht, wobei in der arteriellen oder venösen Schlauchleitung eine Pumpe (9) angeordnet ist, die eine tribo-elektrische Wechselspannung erzeugt, und einer Vorrichtung zur Überwachung eines Zugangs zu dem Patienten, die aufweist:
Mittel (20A) zum Messen einer Wechselspannung und/oder eines Wechselstroms und/oder einer abgeleiteten elektrischen Messgröße, die derart ausgebildet sind,
dass die aufgrund von triboelektrischen Aufladungen beim Betrieb der Pumpe (9) erzeugte elektrische Wechselspannung und/oder der Wechselstrom und/oder die abgeleitete elektrische Messgröße gemessen werden kann, und
Mittel (20B, 20C) zum Auswerten der gemessenen elektrischen Wechselspannung und/oder des Wechselstroms und/oder der abgeleiteten elektrischen Messgröße, die derart ausgebildet sind, dass bei einer Veränderung der Amplitude der gemessenen Wechselspannung und/oder des Wechselstroms und/oder der abgeleiteten elektrischen Messgröße auf einen nicht ordnungsgemäßen Gefäßzugang geschlossen wird, wobei
die Mittel (20A) zum Messen einer Wechselspannung und/oder eines Wechselstroms und/oder einer abgeleiteten elektrischen Messgröße ein erstes elektrisches Kontaktelement (26) und ein zweites elektrisches Kontaktelement (27) aufweisen, zwischen denen die Wechselspannung und/oder der Wechselstrom und/oder die abgeleitete elektrische Größe gemessen wird, wobei das erste Kontaktelement als ein auf die Haut des Patienten aufzulegendes Kontaktelement zur Herstellung einer elektrischen Verbindung mit der Haut des Patienten ausgebildet ist, und das zweite Kontaktelement mit der im Dialysierflüssigkeitskreislauf (II) strömenden Dialysierflüssigkeit und/oder mit dem im Filtratablauf strömenden Filtrat in Kontakt steht.

2. Blutbehandlungsvorrichtung nach Anspruch 1, **dadurch gekennzeichnet, dass** die Mittel (20B, 20C) zum Auswerten der gemessenen elektrischen Wechselspannung und/oder des Wechselstroms und/oder der abgeleiteten elektrischen Messgröße Mittel (20B) zum Vergleichen der Amplitude der Wechselspannung und/oder des Wechselstroms und/oder der abgeleiteten elektrischen Messgröße mit einem vorgegebenen Grenzwert aufweisen, wobei auf einen nicht ordnungsgemäßen Gefäßzugang geschlossen wird, wenn die Amplitude der Wechselspannung und/oder des Wechselstroms und/oder der abgeleiteten elektrischen Messgröße größer als der vorgegebene Grenzwert ist.

3. Blutbehandlungsvorrichtung nach Anspruch 2, **dadurch gekennzeichnet, dass** die Mittel (20B, 20C) zum Vergleichen der Amplitude der Wechselspannung und/oder des Wechselstroms und/oder der abgeleiteten elektrischen Messgröße mit einem vorgegebenen Grenzwert derart ausgebildet sind, dass die Amplitude der Wechselspannung und/oder des Wechselstroms und/oder der abgeleiteten elektrischen Größe zu verschiedenen aufeinanderfolgenden Zeitpunkten mit einem vorgegebenen Grenzwert verglichen werden, wobei auf einen nicht ordnungsgemäßen Gefäßzugang geschlossen wird, wenn für eine vorgegebene Anzahl von Messungen zu verschiedenen aufeinanderfolgenden Zeitpunkten die Amplitude der Wechselspannung und/oder des Wechselstroms und/oder der abgeleiteten elektrischen Größe größer als der vorgegebene Grenzwert ist.

4. Blutbehandlungsvorrichtung nach einem der Ansprüche 1 bis 3, **dadurch gekennzeichnet, dass** die Mittel (20B, 20C) zum Auswerten der gemessenen elektrischen Wechselspannung und/oder des Wechselstroms und/oder der abgeleiteten elektrischen Messgröße Mittel (20C) zum Bestimmen der Frequenz der elektrischen Wechselspannung und/oder des Wechselstroms und/oder der abgeleiteten elektrischen Größe und zum Vergleichen der Frequenz der elektrischen Wechselspannung und/oder des Wechselstroms und/oder der abgeleiteten elektrischen Größe mit der Frequenz des Rotors der Pumpe (9) aufweisen, wobei auf einen nicht ordnungemäßen Gefäßzugang nur dann geschlossen wird, wenn die Frequenz der Wechselspannung und/oder des Wechselstroms und/oder der abgeleiteten elektrischen Größe mit der Frequenz des Rotors der Pumpe korrelieren.

5. Blutbehandlungsvorrichtung nach einem der Ansprüche 1 bis 4, **dadurch gekennzeichnet, dass** die Pumpe (9) eine okkludierende Schlauchpumpe ist.

6. Blutbehandlungsvorrichtung nach einem der Ansprüche 1 bis 5, **dadurch gekennzeichnet, dass** die Blutbehandlungsvorrichtung eine Einrichtung (10) zum Bereitstellen von Dialysierflüssigkeit aufweist, wobei das zweite Kontaktelement (27) in der Einrichtung zur Bereitstellung von Dialysierflüssigkeit mit der Dialysierflüssigkeit in Kontakt steht.

7. Verfahren zur Überwachung des Gefäßzuganges bei einer extrakorporalen Blutbehandlung, mit einem Dialysierflüssigkeitskreislauf (II), der eine Dialysierflüssigkeitskammer (4) eines von einer semipermeablen Membran (2) in eine Blutkammer (3) und die Dialysierflüssigkeitskammer (4) unterteilten Membranfilters (1) einschließt, und/oder einem Filtratablauf, der eine Filtratkammer (4) eines von einer semipermeablen Membran (2) in eine Blutkammer (3), und die Filtratkammer (4) unterteilten Membranfilters (1) einschließt, und einem Blutkreislauf (I), der die Blutkammer des Dialysators einschließt, wobei Blut des Patienten über eine arterielle Schlauchleitung, die einen arteriellen Patientenanschluss mit einer arterielle Punktionskanüle aufweist, dem Patienten entnommen und der Blutkammer zugeführt wird und über eine venöse Schlauchleitung, die einen venösen Patientenanschluss mit einer venösen Punktionskanüle aufweist, aus der Blutkammer dem Patienten wieder zugeführt wird, wobei die Flüssigkeit in der arteriellen oder venösen Schlauchleitung mit einer Pumpe gefördert wird, die eine tribo-elektrische Wechselspannung erzeugt,
**dadurch gekennzeichnet, dass**
die aufgrund von triboelektrischen Aufladungen beim Betrieb der Pumpe erzeugte elektrische Wechselspannung und/oder der Wechselstrom und/oder eine abgeleitete elektrische Messgröße gemessen wird, und bei einer Veränderung der Amplitude der gemessenen Wechselspannung und/oder des Wechselstroms und/oder der abgeleiteten elektrischen Messgröße auf einen nicht ordnungsgemäßen Gefäßzugang geschlossen wird, wobei
die Wechselspannung und/oder der Wechselstrom und/oder die abgeleitete elektrische Messgröße zwischen der Haut des Patienten und der im Dialysierflüssigkeitskreislauf (II) strömenden Dialysierflüssigkeit und/oder in dem im Filtratablauf strömenden Filtrat gemessen wird.

8. Verfahren nach Anspruch 7, **dadurch gekennzeichnet, dass** die Amplitude der Wechselspannung und/oder des Wechselstroms und/oder der abgeleiteten elektrischen Messgröße mit einem vorgegebenen Grenzwert verglichen wird, wobei auf einen nicht ordnungsgemäßen Gefäßzugang geschlossen wird, wenn die Amplitude der Wechselspannung und/oder des Wechselstroms und/oder der abgeleiteten elektrischen Größe größer als der vorgegebene Grenzwert ist.

9. Verfahren nach Anspruch 8, **dadurch gekennzeichnet, dass** die Amplitude der Wechselspannung und/oder des Wechselstroms und/oder der abgeleiteten elektrischen Messgröße zu verschiedenen aufeinanderfolgenden Zeitpunkten mit einem vorgegebenen Grenzwert verglichen werden, wobei auf einen nicht ordnungsgemäßen Gefäßzugang geschlossen wird, wenn für eine vorgegebene Anzahl von Messungen zu verschiedenen aufeinanderfolgenden Zeitpunkten die Amplitude der Wechselspannung und/oder des Wechselstroms und/oder der abgeleiteten elektrischen Größe größer als der vorgegebene Grenzwert ist.

10. Verfahren nach einem der Ansprüche 7 bis 9, **dadurch gekennzeichnet, dass** die Frequenz der elektrischen Wechselspannung und/oder des Wechselstroms und/oder der abgeleiteten elektrischen Messgröße mit der Frequenz des Rotors der Pumpe verglichen wird, wobei auf einen nicht ordnungemäßen Gefäßzugang nur dann geschlossen wird, wenn die Frequenz der Wechselspannung und/oder des Wechselstroms und/oder der abgeleiteten elektrischen Größe und die Frequenz des Rotors der Pumpe korrelieren.

11. Verfahren nach einem der Ansprüche 7 bis 10, **dadurch gekennzeichnet, dass** auf die Haut des Patienten ein elektrisches Kontaktelement (26) aufgelegt wird, wobei die Wechselspannung und/oder der Wechselstrom und/oder die abgeleitete elektrische Messgröße zwischen dem auf der Haut des Patienten aufgelegten Kontaktelement und der im Dialysierflüssigkeitskreislauf (II) strömenden Dialysierflüssigkeit und/oder dem im Filtratablauf strömenden Filtrat gemessen wird.

12. Verfahren nach einem der Ansprüche 7 bis 11, **dadurch gekennzeichnet, dass** die Flüssigkeit mit einer okkludierenden Schlauchpumpe (9) gefördert wird.

## Claims

1. Blood treatment device comprising
a dialysis fluid circuit (II), which includes a dialysis fluid chamber (4) of a membrane filter (1) divided by a semi-permeable membrane (2) into a blood chamber (3) and the dialysis fluid chamber (4), and/or a filtrate outlet, which includes a filtrate chamber (4) of a membrane filter (1) divided by a semi-permeable membrane (2) into a blood chamber (3) and the filtrate chamber (4),
a blood circuit (I), which includes the blood chamber of the dialyser, wherein an arterial tubular conduit (6) connected to an arterial puncture cannula (5) leads to the blood chamber of the membrane filter, and a venous tubular conduit (7) connected to a venous puncture cannula (8) leaves the blood chamber of the membrane filter, wherein a pump (9) generating a triboelectrical alternating voltage is arranged in the arterial or venous tubular conduit, and
a device for monitoring a patient entry, comprising:
means (20A) for measuring an alternating voltage and/or an alternating current and/or a derived electrical variable, which are formed such that the AC voltage generated during operation of the pump (9) owing to triboelectrical charges and/or the alternating current and/or the derived electrical variable can be measured, and
means (20B, 20C) for evaluating the measured AC voltage and/or the alternating current and/or the derived electrical variable, which are formed such that incorrect vascular entry is determined upon a change in the amplitude of the measured alternating voltage and/or of the alternating current and/or of the derived electrical variable, wherein
the means (20A) for measuring an alternating voltage and/or an alternating current and/or a derived electrical variable comprise a first electrical contact element (26) and a second electrical contact element (27), between which the alternating voltage and/or the alternating current and/or the derived electrical variable is measured, wherein the first contact element is formed as a contact element to be placed onto the patient's skin to produce an electrical connection to the patient's skin, and the second contact element is in contact with the dialysis fluid flowing in the dialysis fluid circuit (II) and/or with the filtrate flowing in the filtrate outlet.

2. Blood treatment device according to claim 1, **characterised in that** the means (20B, 20C) for evaluating the measured AC voltage and/or the alternating current and/or the derived electrical variable comprise means (20B) for comparing the amplitude of the alternating voltage and/or of the alternating current and/or of the derived electrical variable with a predetermined threshold, incorrect vascular entry being determined if the amplitude of the alternating voltage and/or of the alternating current and/or of the derived electrical variable is greater than the predetermined threshold.

3. Blood treatment device according to claim 2, **characterised in that** the means (20B, 20C) for comparing the amplitude of the alternating voltage and/or of the alternating current and/or of the derived electrical variable with a predetermined threshold are formed such that the amplitude of the alternating voltage and/or of the alternating current and/or of the derived electrical variable are compared at various successive times with a predetermined threshold, incorrect vascular entry being determined if the amplitude of the alternating voltage and/or of the alternating current and/or of the derived electrical variable is greater than the predetermined threshold for a predetermined number of measurements at various successive times.

4. Blood treatment device according to any of claims 1 to 3, **characterised in that** the means (20B, 20C) for evaluating the measured AC voltage and/or the alternating current and/or the derived electrical variable comprise means (20C) for establishing the frequency of the AC voltage and/or of the alternating current and/or of the derived electrical variable and for comparing the frequency of the AC voltage and/or of the alternating current and/or of the derived electrical variable with the frequency of the rotor of the pump (9), incorrect vascular entry being determined only if the frequency of the alternating voltage and/or of the alternating current and/or of the derived electrical variable correlate with the frequency of the rotor of the pump.

5. Blood treatment device according to any of claims 1 to 4, **characterised in that** the pump (9) is an occlusive peristaltic pump.

6. Blood treatment device according to any of claims 1 to 5, **characterised in that** the blood treatment device comprises a dialysis fluid preparation apparatus (10), the second contact element (27) being in contact with the dialysis fluid in the dialysis fluid preparation apparatus.

7. Method for monitoring vascular entry during an extracorporeal blood treatment, comprising a dialysis fluid circuit (II), which includes a dialysis fluid chamber (4) of a membrane filter (1) divided by a semi-permeable membrane (2) into a blood chamber (3) and the dialysis fluid chamber (4), and/or a filtrate outlet, which includes a filtrate chamber (4) of a membrane filter (1) divided by a semi-permeable membrane (2) into a blood chamber (3) and the filtrate chamber (4), and a blood circuit (I), which includes the blood chamber of the dialyser, the patient's blood being withdrawn from the patient and fed to the blood chamber via an arterial tubular conduit that comprises an arterial patient connector with an arterial puncture cannula and being returned from the blood chamber to the patient via a venous tubular conduit that comprises a venous patient connector with a venous puncture cannula, the fluid being conveyed in the arterial or venous tubular conduit by a pump generating a triboelectrical alternating current,
**characterised in that**
the AC voltage generated during operation of the pump owing to triboelectrical charges and/or the alternating current and/or a derived electrical variable is measured, and incorrect vascular entry is determined upon a change in the amplitude of the measured alternating voltage and/or of the alternating current and/or of the derived electrical variable,
the alternating voltage and/or the alternating current and/or the derived electrical variable being measured between the patient's skin and the dialysis fluid flowing in the dialysis fluid circuit (II) and/or in the filtrate flowing in the filtrate outlet.

8. Method according to claim 7, **characterised in that** the amplitude of the alternating voltage and/or of the alternating current and/or of the derived electrical variable is compared with a predetermined threshold, incorrect vascular entry being determined if the amplitude of the alternating voltage and/or of the alternating current and/or of the derived electrical variable is greater than the predetermined threshold.

9. Method according to claim 8, **characterised in that** the amplitude of the alternating voltage and/or of the alternating current and/or of the derived electrical voltage is compared at various successive times with a predetermined threshold, incorrect vascular entry being determined if the amplitude of the alternating voltage and/or of the alternating current and/or of the derived electrical variable is greater than the predetermined threshold for a predetermined number of measurements at various successive times.

10. Method according to any of claims 7 to 9, **characterised in that** the frequency of the AC voltage and/or of the alternating current and/or of the derived electrical variable is compared with the frequency of the rotor of the pump, incorrect vascular entry being determined only if the frequency of the alternating voltage and/or of the alternating current and/or of the derived electrical variable correlates with the frequency of the rotor of the pump.

11. Method according to any of claims 7 to 10, **characterised in that** an electrical contact element (26) is placed onto the patient's skin, the alternating voltage and/or the alternating current and/or the derived electrical variable being measured between the contact element placed on the patient's skin and the dialysis fluid flowing in the dialysis fluid circuit (II) and/or the filtrate flowing in the filtrate outlet.

12. Method according to any of claims 7 to 11, **characterised in that** the fluid is conveyed using an occlusive peristaltic pump (9).

## Revendications

1. Dispositif de traitement de sang comportant
un circuit de liquide de dialyse (II) qui comprend une chambre de liquide de dialyse (4) d'un filtre à membrane (1) subdivisé par une membrane semi-perméable (2) en une chambre à sang (3) et la chambre de liquide de dialyse (4) et/ou une évacuation de filtrat, qui comprend une chambre de filtrat (4) d'un filtre à membrane (1) subdivisé par une membrane semi-perméable (2) en une chambre à sang (3) et la chambre de filtrat (4),
un circuit sanguin (I) qui comprend la chambre à sang du dialyseur, dans lequel une conduite souple artérielle (6) qui est raccordée à une canule de ponction artérielle (5), conduit à la chambre à sang du filtre à membrane et une conduite souple veineuse (7) qui est raccordée à une canule de ponction veineuse (8), à partir de la chambre à sang du filtre à membrane, dans lequel dans la conduite souple artérielle ou veineuse est agencée une pompe (9) qui génère une tension alternative tribo-électrique, et un dispositif de surveillance d'un accès au patient, qui comporte :
des moyens (20A) de mesure d'une tension alternative et/ou d'un courant alternatif et/ou d'une valeur de mesure électrique dérivée, qui sont conçus de telle sorte que sur la base de charges triboélectriques lors du fonctionnement de la pompe (9), la tension alternative électrique générée et/ou le courant alternatif et/ou les valeurs de mesure électriques dérivées peuvent être mesurés, et
des moyens (20B, 20C) d'évaluation de la tension alternative électrique mesurée et/ou du courant alternatif et/ou de la valeur de mesure électrique dérivée, qui sont conçus de telle sorte que lors d'une modification de l'amplitude de la tension alternative mesurée et/ou du courant alternatif et/ou de la valeur de mesure électrique dérivée, on en déduit qu'un accès vasculaire n'est pas normal, dans lequel
les moyens (20A) de mesure d'une tension alternative et/ou d'un courant alternatif et/ou d'une valeur de mesure électrique dérivée présentent un premier élément de contact électrique (26) et un deuxième élément de contact électrique (27) entre lesquels la tension alternative et/ou le courant alternatif et/ou la valeur de mesure électrique dérivée sont mesurés, le premier élément de contact étant conçu comme un élément de contact à placer sur la peau du patient pour la production d'une liaison électrique avec la peau du patient, et le deuxième élément de contact étant en contact avec le liquide de dialyse s'écoulant dans le circuit de liquide de dialyse (II) et/ou avec le filtrat s'écoulant dans l'évacuation de filtrat.

2. Dispositif de traitement de sang selon la revendication 1, **caractérisé en ce que** les moyens (20B, 20C) pour évaluer la tension électrique alternative mesurée et/ou le courant alternatif et/ou la valeur de mesure électrique dérivée présentent des moyens (20B) pour comparer l'amplitude de la tension alternative et/ou du courant alternatif et/ou de la valeur électrique dérivée avec une valeur seuil prédéterminée, dans lequel on déduit qu'un accès vasculaire n'est pas normal lorsque l'amplitude de la tension alternative et/ou du courant alternatif et/ou de la valeur de mesure électrique dérivée est supérieure à la valeur seuil prédéterminée.

3. Dispositif de traitement de sang selon la revendication 2, **caractérisé en ce que** les moyens (20B, 20C) de comparaison de l'amplitude de la tension alternative et/ou du courant alternatif et/ou de la valeur de mesure électrique dérivée sont conçus avec une valeur seuil prédéterminée, de telle sorte que l'amplitude de la tension alternative et/ou du courant alternatif et/ou de la valeur de mesure électrique dérivée à différents moments consécutifs est comparée à une valeur seuil prédéterminée, dans lequel on déduit qu'un accès vasculaire n'est pas normal lorsque, pour un nombre de mesures prédéterminé, à différents moments consécutifs, l'amplitude de la tension alternative et/ou du courant alternatif et/ou de la valeur de mesure électrique dérivée est supérieure à la valeur seuil prédéterminée.

4. Dispositif de traitement de sang selon l'une des revendications 1 à 3, **caractérisé en ce que** les moyens (20B, 20C) d'évaluation de la tension alternative électrique mesurée et/ou du courant alternatif et/ou de la valeur de mesure électrique dérivée présentent des moyens (20C) de détermination de la fréquence de la tension alternative électrique et/ou du courant alternatif et/ou de la valeur électrique dérivée, et de comparaison de la fréquence de la tension alternative électrique et/ou du courant alternatif et/ou de la valeur électrique dérivée avec la fréquence du rotor de la pompe (9), dans lequel on déduit qu'un accès vasculaire n'est pas normal, uniquement lorsque la fréquence de la tension alternative et/ou du courant alternatif et/ou de la valeur électrique dérivée sont en corrélation avec la fréquence du rotor de la pompe.

5. Dispositif de traitement de sang selon l'une des revendications 1 à 4, **caractérisé en ce que** la pompe (9) est une pompe péristaltique occlusive.

6. Dispositif de traitement de sang selon l'une des revendications 1 à 5, **caractérisé en ce que** le dispositif de traitement de sang présente un système (10) pour la mise à disposition du liquide de dialyse, dans lequel le deuxième élément de contact (27) du système de mise à disposition du liquide de dialyse est en contact avec le liquide de dialyse.

7. Procédé de surveillance de l'accès vasculaire dans un traitement de sang extracorporel comportant un circuit de liquide de dialyse (II) qui comprend une chambre de liquide de dialyse (4) d'un filtre à membrane (1) subdivisé par une membrane semi-perméable (2) en une chambre à sang (3) et la chambre de liquide de dialyse (4) et/ou une évacuation de filtrat, qui comprend une chambre de filtrat (4) d'un filtre à membrane (1) subdivisé par une membrane semi-perméable (2) en une chambre à sang (3) et la chambre de filtrat (4), et un circuit sanguin (I) qui comprend la chambre à sang du dialyseur, dans lequel le sang du patient est retiré du patient par une conduite souple artérielle qui présente un raccord artériel au patient doté d'une canule de ponction artérielle et est conduit à la chambre à sang et est réacheminé au patient à partir de la chambre à sang par une conduite souple veineuse qui présente un raccord veineux au patient, doté d'une canule de ponction veineuse, dans lequel le fluide dans la conduite souple artérielle ou veineuse est acheminé par une pompe qui génère une tension alternative tribo-électrique,
**caractérisé en ce que**
la tension alternative électrique, générée par les charges triboélectriques lors du fonctionnement de la pompe, et/ou le courant alternatif et/ou une valeur de mesure électrique dérivée sont mesurés et lors d'une modification de l'amplitude de la tension alternative mesurée et/ou du courant alternatif et/ou de la valeur de mesure électrique dérivée, on déduit qu'un accès vasculaire n'est pas normal, dans lequel la tension alternative et/ou le courant alternatif et/ou la valeur de mesure électrique dérivée sont mesurés entre la peau du patient et le filtrat s'écoulant dans le circuit de liquide de dialyse (II) et/ou dans le filtrat s'écoulant dans l'évacuation du filtrat.

8. Procédé selon la revendication 7, **caractérisé en ce que** l'amplitude de la tension alternative et/ou du courant alternatif et/ou de la valeur de mesure électrique dérivée est comparée à une valeur seuil prédéterminée, dans lequel on déduit qu'un accès vasculaire n'est pas normal lorsque l'amplitude de la tension alternative et/ou du courant alternatif et/ou la valeur électrique dérivée est supérieure à la valeur seuil prédéterminée.

9. Procédé selon la revendication 8, **caractérisé en ce que** l'amplitude de la tension alternative et/ou du courant alternatif et/ou de la valeur de mesure électrique dérivée est comparée à différents moments consécutifs à une valeur seuil prédéterminée, dans lequel on déduit qu'un accès vasculaire n'est pas normal lorsque, pour un nombre de mesures données à différents moments consécutifs, l'amplitude de la tension alternative et/ou du courant alternatif et/ou la valeur électrique dérivée est supérieure à la valeur seuil prédéterminée.

10. Procédé selon l'une des revendications 7 à 9, **caractérisé en ce que** la fréquence de la tension alternative électrique et/ou du courant alternatif et/ou de la valeur de mesure électrique dérivée est comparée à la fréquence du rotor de la pompe, dans lequel on déduit qu'un accès vasculaire n'est pas normal, uniquement lorsque la fréquence de la tension alternative et/ou du courant alternatif et/ou de la valeur électrique dérivée et la fréquence du rotor de la pompe sont corrélées.

11. Procédé selon l'une des revendications 7 à 10, **caractérisé en ce que** sur la peau du patient est placé un élément de contact électrique (26), dans lequel la tension alternative et/ou le courant alternatif et/ou la valeur de mesure électrique dérivée sont mesurés entre l'élément de contact placé sur la peau du patient et le liquide de dialyse s'écoulant dans le circuit de liquide de dialyse (II) et/ou le filtrat s'écoulant dans l'évacuation de filtrat.

12. Procédé selon l'une des revendications 7 à 11, **caractérisé en ce que** le liquide est acheminé avec une pompe péristaltique occlusive (9).
